# EUROPÄISCHE PATENTSCHRIFT

(11) **EP 0 979 068 B1**
(45) Veröffentlichungstag und Bekanntmachung des Hinweises auf die Patenterteilung: **07.12.2005**
(21) Anmeldenummer: 98928200.9
(22) Anmeldetag: 16.04.1998
(51) Int. Cl.: A61K 7/50

(54) **WÄSSRIGE KATIONISCHE TENSIDZUBEREITUNGEN, EIN VERFAHREN ZU IHRER HERSTELLUNG SOWIE IHRE VERWENDUNG**
AQUEOUS CATIONIC TENSIDE PREPARATIONS, METHOD FOR THE PRODUCTION AND USE THEREOF
PREPARATIONS TENSIOACTIVES CATIONIQUES AQUEUSES, PROCEDE PERMETTANT DE LES PREPARER ET LEUR UTILISATION

(30) Priorität: 28.04.1997 DE 19717925
(43) Veröffentlichungstag der Anmeldung: 16.02.2000
(73) Patentinhaber: BASF Aktiengesellschaft, 67056 Ludwigshafen (DE)
(72) Erfinder: HÖSSEL, Peter, D-67105 Schifferstadt (DE); ZIRNSTEIN, Michael, D-69198 Schriesheim (DE); SCHUNTER, Walter, D-67251 Freinsheim (DE); WIRSING, Friedrich, D-67346 Speyer (DE); KASEL, Wolfgang, D-69226 Nu loch (DE); OPPENLÄNDER, Knut, D-67061 Ludwigshafen (DE)
(86) Internationale Anmeldenummer: PCT/EP1998/002241
(87) Internationale Veröffentlichungsnummer: WO 1998/048778

(56) Entgegenhaltungen:
- DE-A- 19 548 660
- GB-A- 2 196 980
- US-A- 4 976 956

## Beschreibung

Die vorliegende Erfindung betrifft wässrige kationische Tensidzubereitungen, ein Verfahren zu ihrer Herstellung sowie ihre Verwendung.

Kationische Tenside sind gesuchte Verbindungen, die für viele Anwendungen gebraucht werden und in einer Vielzahl von Publikationen und Patenten beschrieben werden. So werden beispielsweise in DE/OS 34 17 646 und DE/OS 27 10 468 quartäre Ammoniumverbindungen als wichtigste Vertreter der kationischen Tenside wie Cetyltrimethylammoniumchlorid oder Stearyltrimethylammoniumchlorid für die Verwendung in Haarnachbehandlungsmitteln beschrieben. Von Nach teil bei der Verwendung dieser kationischen Tenside ist, daß diese Tenside eine starke korrosive Wirkung aufweisen.

EP-B-0 098 802 beschreibt die Herstellung von quartären Ammoniumverbindungen wie beispielsweise Cetyl-Dimethyl-(2)-Hydroxy-Ethylammoniumdihydrogenphosphat mit Ethylenoxid in Gegenwart von Säuren bevorzugt in Gegenwart von Phosphorsäure und die Verwendung dieser kationischen Tenside in Haarkosmetika. Diese Tenside ebenso wie die oben genannten Verbindungen frieren leicht aus den Lösungen aus. Sie müssen deshalb vor der Anwendung gelöst und homogenisiert werden. Oder aber sie müssen in speziellen Lagertanks so gelagert werden, daß sie nicht ausfrieren können. Beides verursacht zusätzliche Kosten.

In DE/OS 31 16 087 wird ein Verfahren zur Herstellung von quartären Ammoniumverbindungen aus einem tertiären Amin und Epoxiden in Gegenwart von quartären Ammoniumverbindungen als Katalysator beschrieben. Auf diesem Wege lassen sich kationische Tenside wie 2-Hydroxy-hexadecyl-2-hydzoxyethyldimethylammoniumchlorid herstellen. Auch diese Verbindungen frieren leicht aus und wirken korrosiv.

Weiterer Stand der Technik kann den Dokumenten US-A-4 976 956, DE-A-19 548 660 und GB-A-2 196 980 entnommen werden.

Kationische Tenside sollten eine Reihe von vorteilhaften Eigenschaften aufweisen. Wichtige Anforderungen an derartige Tenside sind beispielsweise
1. gute Solubilisatorwirkung,
2. gute Aniontensidverträglichkeit,
3. gute Schaumstabilisatorwirkung,
4. keine oder geringe korrosive Wirkung,
5. gute biozide Wirkung,
6. gute Lagerstäbilität,
7. kein Ausfrieren der Tenside aus der Tensidlösung,
8. kein Halogen im Tensid,
9. gute Konditionierwirkung,
10. gutes Netz-, Emulgier- und Dispergiervermögen,
11 einfache Herstellung.

Aufgabe der vorliegenden Erfindung war es, ein kationisches Tensid zu entwickeln, das möglichst eine Vielzahl der vorteilhaften Eigenschaften erfüllt und die Nachteile der bisher bekannten kationischen Tenside nicht aufweist. Die Aufgabe wurde durch die erfindungsgemäßen wässrigen kationische Tensidzubereitungen, enthaltend
a) 10 bis 50 Gew.-% der quartären Ammoniumverbindung N,N,N-Trimethyl-C₁₂-C₁₄-Alkyl-Ammoniummethosulfat,
b) 0,01 bis 15 Gew.-% des Amins N,N-Dimethyl-C₁₂-C₁₄-Alkyl-Amin und
c) 0,1 bis 5,0 Gew.-% eines Puffers, der in einem Bereich von pH 4 bis 9 puffert,
gelöst.

Die C₁₂-C₁₄-Alkyl-Reste haben vorteilhafterweise folgende Bedeutung:
n-Dodecyl, n-Tridecyl, n-Tetradecyl, 3-Ethyl-5-isopropyl-4-methyloctyl-,
   oder verzweigte oder unverzweigte ungesättigte Alkylketten wie beispielsweise Dodecenyl, Tridecenyl, Tetradecenyl, bevorzugt werden gesättigte und ungesättigte Alkyl- oder Alkenylreste, die sich von aus Fettalkoholen wie Capronalkohol, Önanthalkohol, Caprylalkohol, Pelargonalkohol, Caprinalkohol, Laurylalkohol, Myristylalkohol, Pentadecanol, Cetylalkohol oder aus linearen Ziegler-Alkoholen abgeleiteten Aminen (Aminierung der Alkohole zum Amin) herleiten lassen, besonders bevorzugt sind gesättigte C₁₂-C₁₄-Alkylketten, die sich beispielsweise auf Basis von Kokosfett oder Palmkernöl herstellen lassen. Vorteilhafte verzweigte Alkylreste können beispielsweise auf Basis von Alkoholen aus der Guerbet- oder Oxoreaktion dargestellt werden.

Die quartären Ammoniumverbindungen (a) sind einem Bereich von 10 bis 50 Gew.-% bevorzugt von 15 bis 40 Gew.-%, besonders bevorzugt von 20 bis 35 Gew.-% bezogen auf das Gesamtgewicht in der Tensidzubereitung enthalten.

Die Amine (b) sind in einem Bereich von 0,01 bis 15 Gew.-%, bevorzugt von 0,1 bis 10 Gew.-%, besonders bevorzugt von 0,5 bis 5 Gew.-% bezogen auf das Gesamtgewicht in der Tensidzubereitung enthalten.

Es wurde gefunden, daß durch Zugabe des Puffers zur Tensidlösung die Lagerstabilität der Tensidlösung, ohne die Wirkung des Tensids negativ zu beeinflussen, deutlich verbessert werden konnte. Darüberhinaus konnte die Solubilisatorwirkung der Tensidlösung durch Zugabe des Puffers ebenfalls deutlich verbessert werden.

Die erfindungsgemäßen Tensidzubereitungen enthalten vorteilhafterweise 0,1 bis 5,0 Gew.-%, bevorzugt 0,5 bis 4 Gew.-%, besonders bevorzugt 1,0 bis 3,5 Gew.-% Puffer (c) bezogen auf das Gesamtgewicht der Tensidzubereitung.

Für die erfindungsgemäßen wässrigen kationischen Tensidzubereitungen eignen sich prinzipiell alle bekannten Puffer, die in einem pH-Wertbereich von pH 4 bis 9, bevorzugt von 5 bis 8, besonders bevorzugt von 6 bis 8 puffern, wie beispielsweise im Handbook of Biochemistry (Es. Sober, H.A., Horte R.A., The Chemical Rubber Co., 1968: J-195 - J-199) beschrieben.

Geeignete Puffer sind beispielsweise alle Salze aus schwachen Säuren und starken Basen oder starken Säuren und schwachen Basen, wobei es sich um Salze derselben Säuren und Basen oder Mischungen aus unterschiedlichen Säuren oder Basen handeln kann.

Als Puffer kommen beispielsweise Puffer wie Walpole-Puffer (Essigsäure/NaAcetat), Gomori-Aconitat-Puffer (Aconitinsäure/NaOH), Kolthoff-Puffer (Borax/Succinat), Sörensen's Citrat II-Puffer (Dinatriumcitrat/NaOH), McIlvaine's Citronensäure/Phosphat-Puffer (Citronensäure/Dinatriumphosphat), Stafford, Watson und Rand's Dimethylglutarsäure-Puffer (Dimethylglutarsäure/NaOH), Sörensen's Phosphat-Puffer (Kaliumdihydrogenphospat/Dinatriumhydrogenphosphat), Gomori-Trismaleat-Puffer (Trismaleat/NaOH) oder Gomori-Succinat-Puffer (Succinat/NaOH) in Frage. Auch Puffer wie MES, ADA, PIPES, BIS-TRIS, MOPSO, BIS-TRIS PROPANE, MOPS, DIPSO, TAPSO, HEPPSO, POPSO, EPPS, TEA, TAPS oder ACES, die in der Biochemie übliche Puffer sind, oder Aminosäurepuffer sind geeignete Puffer. Bevorzugt werden Puffer, die sich vorteilhafterweise aus schwachen Säuren und ihren Salzen herstellen lassen, wie beispielsweise Natriumacetat/Essigsäure, Natriumcitrat/Citronensäure, Natriumborat/Borsäure, Natriumphosphat/Phosphorsäure, Kaliumphosphat/Phosphorsäure, Hydrogencarbonat/Soda, Natriumhydroxid/Citronensäure, Natriumhydroxid/Weinsäure oder deren Mischungen. Auch Puffer wie Cholaminchlorid, BES, TES, HEPES, Acetamidoglycin, Glycinamid, Tris, Bicine, Tricine, Glycylglycin oder Puffer auf Basis Ethanolamin oder Diethanolamin sind geeignete Puffer.

Es können einzelne Puffer oder Mischungen in der erfindungsgemäßen Tensidzubereitung verwendet werden.

Besonders bevorzugt werden Puffer oder Puffermischungen auf Basis Hydrogenphosphat/Dihydrogenphospat wie Natriumphosphat/Phosphorsäure, Kaliumphosphat/Phosphorsäure, Dinatriumhydrogenphosphat/Natriumdihydrogenphosphat, Dikaliumhydrogenphosphat/Kaliumdihydrogenphosphat oder KOH/H₃PO₄ oder NaOH/H₃PO₄ verwendet, da sie neben den oben genannten vorteilhaften Wirkungen bekanntermaßen eine Korrosionsschutzwirkung haben.

Die erfindungsgemäßen Tensidzubereitungen weisen außerdem als vorteilhafte Eigenschaft eine ausgezeichnete Kältelagerstabilität auf, das heißt die Zubereitungen frieren nicht unterhalb von 20°C aus. Sie zeigen unterhalb dieser Temperatur im Gegensatz zu den am Markt befindlichen Produkten keine Ausfällungen bzw. Inhomogenitäten. Dies ist einerseits auf die bevorzugte Länge der längeren Alkylketten von C₈-C₁₆, wobei der Anteil der C₁₆-Alkylkette in in der Alkylkettenverteilung möglichst gering sein sollte, und andererseits auf den Puffer zurückzuführen. Bei hohen C₁₆-Alkylkettenanteilen kann die Kältestabilität der erfindungsgemäßen Tensidzubereitungen abnehmen.

Die erfindungsgemäßen Tensidzubereitungen können gegebenenfalls dem Fachmann bekannte weitere Zusatz- oder Hilfsstoffe wie Elektrolyten zur Viskositätserhöhung, Kochsalz oder andere übliche Salze, Hydroxyalkylcellulose, Kationische Cellulosen, kationische synthetische Polymere, andere kationische Tenside wie die Dehyquart®-Marken oder Luvipuat®-Marken, Alkylethersulfate oder Alkylpolyglycoside enthalten oder mit weiteren Tensiden beispielsweise mit kationischen oder anionischen Tensiden kombiniert werden.

Die erfindungsgemäßen Tensidzubereitungen können auf einer Reihe dem Fachmann bekannten Wegen synthetisiert werden beispielsweise unter Verwendung von Alkylierungsmitteln wie Alkylsulfaten, Alkylsulfonaten, Alkylhalogenide oder anderen Aktivestern ausgehend von unterschiedlichen Aminen als Ausgangssubstanzen, vorteilhafterweise wird das erfindungsgemäße verfahren zur Synthese verwendet.

Das gefundene erfindungsgemäße Verfahren zur Herstellung von Verbindungen der Formel I ist, dadurch gekennzeichnet, daß man Amine der allgemeinen Formel III 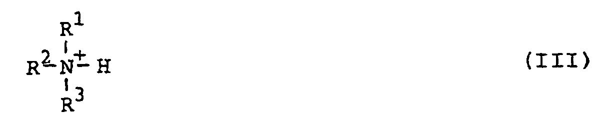 in wässriger Lösung mit einem Dialkylsulfat der allgemeinen Formel IV 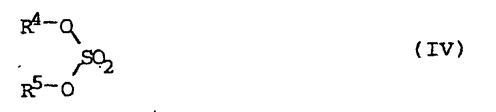 wobei die Substituenten R¹ bis R⁵ die oben genannte Bedeutung haben, in einem Molverhältnisbereich von 85 bis 99,99 Mol% bezogen auf das Amin bei einer Temperatur von 20 bis 100°C umsetzt und anschließend zur Reaktionslösung 0,1 bis 5,0 Gew.-% eines Puffers gibt, der in einem Bereich von pH 4 bis 9 puffert.

Vorteilhafterweise wird im erfindungsgemäßen Verfahren das Dialkylsulfat in einem Molverhältnis von 85 bis 99,9 Mol%, bevorzugt in einem Bereich von 95 bis 99,5 Mol%, besonders bevorzugt in einem Bereich von 97,0 bis 99,0 Mol% bezogen auf das Amin (= 100 Mol%) verwendet, so daß immer ein Überschuß an Amin vorhanden ist und daß das bei der Reaktion entstehende Monoalkylsulfat maximal in äquimolaren Mengen zur entstandenen quartären Ammoniumverbindung in der Tensidzubereitung vorhanden ist.

Die Reaktion wird in einem vorteilhaften Temperaturbereich von 20 bis 100°C, bevorzugt von 40 bis 90°C, besonders bevorzugt von 50 bis 80°C durchgeführt.

Die Alkylierung kann unter pH-Kontrolle, etwa durch Zugabe von Natron- oder Kalilauge, oder ohne pH-Kontrolle durchgeführt werden. Bevorzugt findet keine pH-Kontrolle statt. Die bei pH-Steuerung zugegebene Natron- öder Kalilauge kann später vorteilhaft zu einem Bestandteil des Puffers werden.

Im erfindungsgemäßen Verfahren wird vorteilhaft eine wässrige Alkylaminlösung, -emulsion oder -suspension vorgelegt und mit dem zudosierten Dialkylsulfat zur Reaktion gebracht. Die Zudosierung des Dialkylsulfats wird dabei vorteilhafterweise so gesteuert, daß die oben genannten Reaktionstemperaturbereiche nicht überschritten werden. Prinzipiell kann auch das Dialkylsulfat vorgelegt werden und das Alkylamin zudosiert werden oder die Reaktion in einer Mischkammer bei gleichzeitiger Zugabe der Reaktanten durchgeführt werden. Vorteilhafterweise wird die Reaktion, um eine rasche Zugabe des Dialkylsulfats zu ermöglichen, gekühlt und unter inerten Bedingungen durchgeführt, wobei eine Kühlung jedoch in der Regel nicht notwendig ist.

Nach Beendigung der Alkylierung kann der Puffer in einer bevorzugten Ausführungsform zur Reaktionslösung gegeben werden, wobei der Puffer in der Lösung wie bevorzugt erzeugt werden kann oder aber als fertiger Puffer der Reaktionslösung zugesetzt werden kann. Es können Puffer als Lösungen oder als Festsubstanzen verwendet werden. Der Puffer kann prinzipiell vor, während oder nach der Alkylierungsreaktion zugegeben werden, bevorzugt wird der Puffer nach Beendigung der Reaktion zugegeben.

Die erfindungsgemäßen kationischen Tensidzubereitungen eignen sich für eine Reihe von Anwendungsgebieten wie beispielsweise bevorzugt für Kosmetika wie kosmetische Formulierungen mit Pflege- und/oder Konditioniereigenschaften beispielsweise Stylingprodukte wie Haarschäume, Haargele, Haarsprays oder Nachbehandlungsmittel wie Haarwasser, Lotionen, Kurspülungen, Kurpackungen, Spitzenfluids, Hair-Repair-Mittel, "Hot Oil Treatments", Shampoos, Flüssigseifen oder Pflegecremes.

Die Tensidzubereitungen eignen sich als Hydrophobiermittel, als Antistatikum, zur Beschichtung von Textilien oder Leder, als Galvanikhilfsmittel, als Hilfsstoff zur Metallreinigung, als Korrosionsinhibitor, als Flockungs- oder Koagulationsmittel, als Hilfsmittel für den Textildruck, für Druckfarben und Druckplatten, als Dispergiermittel für Farben und Lacke, als Hilfsmittel für die Elektro- und Elektronikindustrie, als Biozid bzw. als Bestandteil von Desinfektionsmitteln, als Pharmahilfsmittel oder für die Tierkosmetik. Darüberhinaus sind die erfindungsgemäßen Tensidzubereitungen als Polymerisationsstarter für kationische Polymerisationen, als Polymerisationsmittel oder als Bindemittel von Bitumen geeignet.

### Beispiele

### Beispiel 1: Herstellung der kationischen Tensidzubereitung

In einem Rührreaktor wurden 72,0 kg vollentsalztes Wasser und 25,0 kg N,N-Dimethylkokosamin (70 - 75 % Dodecyl, 23 - 28 % Tetradecyl, 0 - 2 % Decyl, 0 - 2 % Hexadecyl; Aminzahl 4,22 - 4,55 mmol basisch N/g) zur Herstellung von N,N,N-Trimethyl-kokosalkylammoniummethosulfat (INSI-Name: Cocotrimonium Methosulfat) vorgelgt. Unter Rühren wurden 13,9 kg Dimethylsulfat bei 65 - 70°C unter Schutzgas so zugegeben, daß die Temperatur gehalten werden konnte. Nach Abkühlen auf 50°C wurden 1,40 kg 85 %ige Phosphorsäure, 1,70 kg 50 %ige Kalilauge sowie 14,3 kg vollentsalztes Wasser hinzugegeben.
Austrag 126 kg wässrige Produktlösung mit einer Gardner-Farbzahl <1,
Dimethylsulfat unter Nachweisgrenze;
Nichtflüchtiger Anteil: 31 %
Basischer Stickstoff: 0,07 %

### Beispiel 2: Anwendungstechnische Vergleichsprüfung verschiedener Produkte mit kationischen Tensiden

Die wie unter Beispiel 1 beschrieben, einfach hergestellbaren Tensidzubereitungen zeigten eine Reihe von anwendungstechnischen Vorteilen wie gute Kältelagerstabilität, daß heißt 25 bis 30 %ige Tensidlösungen zeigten unterhalb von 20°C keine Ausfällungen oder Inhomogenitäten. Ein Erwärmen der Tensidlösung vor der Anwendung und/oder ein mechanisches Durchrühren der Lösung, um wieder ein klare Lösung zu erhalten, entfällt. Daneben sind die unter Beispiel 1 hergestellten Lösungen nicht korrosiv, zeigen gute Solubilisatorwirkung und sind insgesamt lagerstabil. Auch über eine längere Lagerung (mehrere Wochen) verändern sich die Tensidlösungen nicht. Diese vorteilhaften Eigenschaften sind auf die Kombination der speziellen quartären Ammoniumverbindungen, dem Amin und dem Puffer zurückzuführen.

Tabelle 1 zeigt einen Vergleich zwischen den erfindungsgemäßen Tensidzubereitungen und verschiedenen Markprodukten in dem diese vorteilhaften Eigenschaften klar zum Ausdruck kommen. Alle Vergleichsprodukten Luviquat® Mono CP (Cetylhydroxyethyldimethylammoniumdihydrogenphosphat), Dehyquart® A (Cetyltrimethylammoniumchlorid), Dehyquart® E [N-(2-Hydroxyhexadecyl-1)-N,N-dimethyl-N-2-hydroxyethylammoniumchlorid], Dehyquart® SP (Trisoligooxyethylalkylammoniumphosphat) besitzen Nachteile bezüglich der Kältelagerstabilität gegenüber den erfindungsgemäßen wie unter Beispiel 1 hergestellten Tensidzubereitungen (Luviquat® Mono LS = N,N,N-Trimethyl-C₁₂/C₁₄-ammoniummethylsulfat, siehe Tabelle 1).

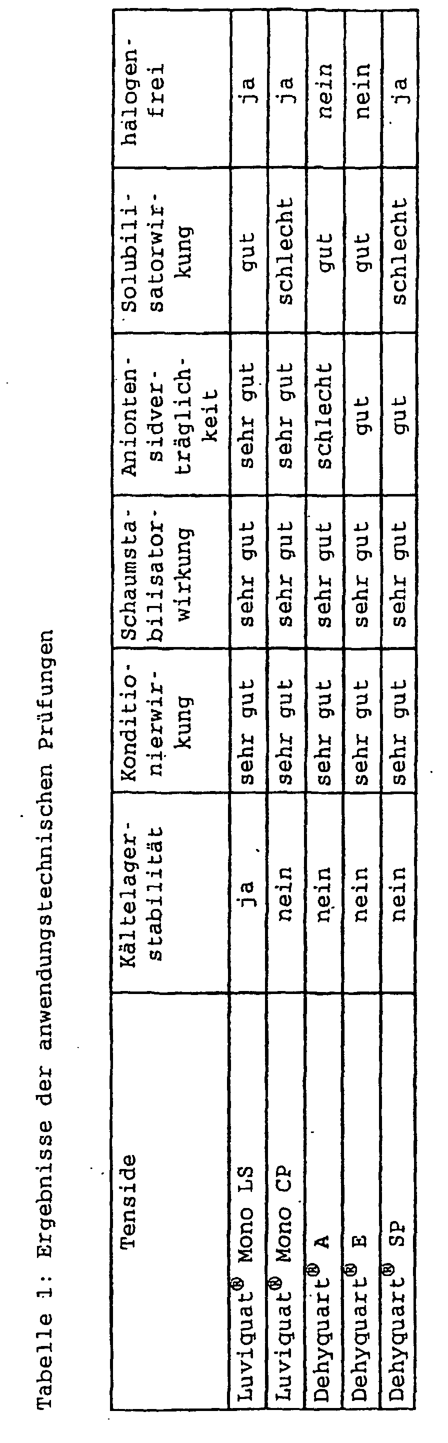

Die in Tabelle 1 angegebenen Eigenschaften wurden nach folgenden Methoden ermittelt.
- Kältelagerstabilität:
   Zur Ermittelung der Kältelagerstabilität wurden die verschiedenen Tensidlösungen in einem Langzeittest ein Jahr bei 6 °C gelagert und anschließend auf eventuelle Trübungen und Ausfällungen hin untersucht. In einem Schnelltest wurden die Proben von 20 °C auf - 18 °C abgekühlt und wieder aufgetaut und anschließend wieder auf eventuelle Trübungen und Ausfällungen hin untersucht.
- Konditionierwirkung:
   Die Konditionierwirkung wurde in einer subjektiven Prüfung der Naßkämmbarkeit von Testhaarsträhnen ermittelt. Dazu wurden die Haarsträhnen mit den kationischen Tensidlösungen oder -zubereitungen behandelt und anschließend die Kämmbarkeit von drei unabhängigen Personen mit sehr gut, gut oder schlecht beurteilt.
- Schaumstabilisatorwirkung:
   Für die Ermittelung der Schaumstabilisatorwirkung wurden mit den Tensiden Schäume erzeugt, die Schaumqualität beurteilt und die Stabilität der Schäume über die Zeit gemessen.
- Aniontensidverträglichkeit:
   Die Aniontensidverträglichkeit wurde in einer 4 %igen Mischung der Tenside (Wirkstoffgehalt) mit 50 % Texapon NSO (= 28 % Natriumlaurylethersulfat in Wasser) getestet.
   Eine klare Lösung der Mischung wurde mit sehr gut, eine leichte Trübung mit gut und Ausfällungen in der Mischung mit schlecht beurteilt.
- Solubilisatorwirkung:
   Die Solubilisatorwirkung wurde mit neun verschiedenen in der Kosmetik üblichen Ölen getestet (siehe Tabelle 2). Die Wirkung der Tenside wurde nach 24 Stunden beurteilt. Ab sechs solubilisierten Ölen wurde ein gute Beurteilung vergeben.

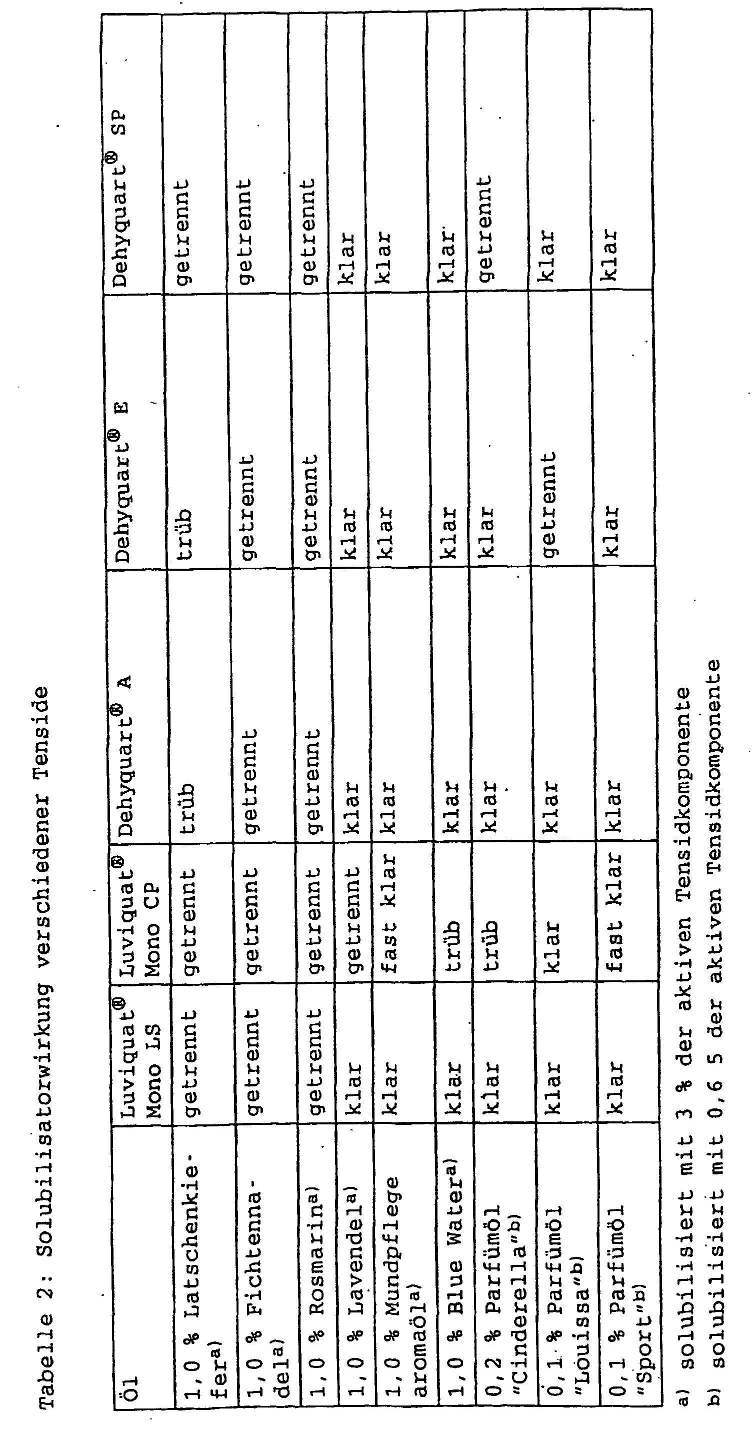

## Patentansprüche

1. Wässrige kationische Tensidzubereitungen, enthaltend
a) 10 bis 50 Gew.-% der quartären Ammoniumverbindung N,N,N-Trimethyl-C₁₂-C₁₄-Alkyl-Ammoniummethosulfat,
b) 0,01 bis 15 Gew.-% des Amins N,N-Dimethyl-C₁₂-C₁₄-Alkyl-Amin und
c) 0,1 bis 5,0 Gew.-% eines Puffers, der in einem Bereich von pH 4 bis 9 puffert.

2. Tensidzubereitungen nach Anspruch 1 enthaltend als Puffer einen Hydrogen/Dihydrogenphosphatpuffer.

3. Verfahren zur Herstellung von Tensidzubereitungen gemäß Anspruch 1, **dadurch gekennzeichnet, daß** man N,N-Dimethyl-C₁₂-C₁₄-Alkyl-Amin in wässriger Lösung mit einem Dialkylsulfat der allgemeinen Formel IV 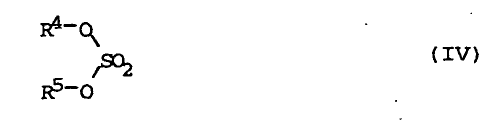 wobei die Substituenten R⁴ und R⁵ Methyl bedeuten, in einem Molverhältnisbereich von 85 bis 99,99 Mol% bezogen auf das Amin bei einer Temperatur von 20 bis 100 °C umsetzt und anschließend zur Reaktionslösung 0,1 bis 5,0 Gew.-% eines Puffers gibt, der in einem Bereich von pH 4 bis 9 puffert.

4. Verwendung einer Tensidzubereitung gemäß den Ansprüchen 1 bis 2 in der Kosmetik.

5. Verwendung einer Tensidzubereitung gemäß den Ansprüchen 1 bis 2 in Haarkosmetika.

6. Verwendung einer Tensidzubereitung gemäß den Ansprüchen 1 bis 2 in Haarfestigern, Haarspülungen, Haarsprays, in Dauerwellmitteln oder in Haarfärbemitteln.

## Claims

1. An aqueous cationic surfactant formulation comprising
a) from 10 to 50% by weight of the quaternary ammonium compound N,N,N-trimethyl-C₁₂-C₁₄-alkylammonium methosulfate,
b) from 0.01 to 15 % by weight of the amine N,N-dimethyl-C₁₂-C₁₄-alkylamine, and
c) from 0.1 to 5.0 % by weight of a buffer which maintains a pH in a range from 4 to 9.

2. The surfactant formulation according to claim 1, comprising as buffer a hydrogen/dihydrogen phosphate buffer.

3. A process for preparing a surfactant formulation according to claim 1, which comprises reacting
N,N-dimethyl-C₁₂-C₁₄-alkylamine in aqueous solution with a dialkyl sulfate of the formula IV 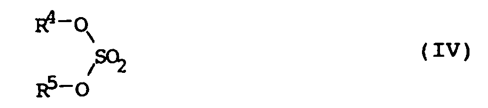 where R⁴ and R⁵ are methyl, in a molar ratio range from 85 to 99.99 mol%, based on the amine and at from 20 to 100°C and then adding to the reaction solution from 0.1 to 5.0% by weight of a buffer which maintains a pH in a range from 4 to 9.

4. The use of a surfactant formulation according to either of claims 1 and 2 in cosmetology.

5. The use of a surfactant formulation according to either of claims 1 and 2 in hair cosmetics.

6. The use of a surfactant formulation according to either of claims 1 and 2 in hairsetting agents, hair rinses, hair sprays, permanent waving compositions or hair coloring compositions.

## Revendications

1. Compositions tensioactives cationiques aqueuses, contenant
a) 10 à 50 % en poids du composé d'ammonium quaternaire, le méthosulfate de N,N,N-triméthyl-C₁₂-C₁₄-alkyl-ammonium,
b) 0,01 à 15 % en poids de l'amine, la N,N-diméthyl-C₁₂-C₁₄-alkylamine, et
c) 0,1 à 5,0 % en poids d'un tampon qui tamponne dans une gamme de pH de 4 à 9.

2. Compositions tensioactives suivant la revendication 1, contenant, comme tampon, un tampon d'hydrogène/dihydrogénophosphate.

3. Procédé de préparation de compositions tensioactives suivant la revendication 1, **caractérisé en ce que**, à une température de 20 à 100°C, on fait réagir de la N,N-diméthyl-C₁₂-C₁₄-alkylamine en solution aqueuse avec un sulfate de dialkyle de la formule générale IV : 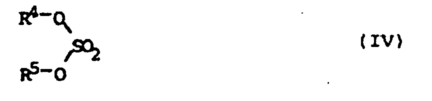 dans laquelle les substituants R⁴ et R⁵ représentent du méthyle, dans une gamme de proportions molaires de 85 à 99,99 % molaires par rapport à l'amine, et ensuite on introduit dans la solution réactionnelle 0,1 à 5,0 % en poids d'un tampon qui tamponne dans une gamme de pH de 4 à 9.

4. Utilisation d'une composition tensioactive suivant les revendications 1 et 2, en cosmétique.

5. Utilisation d'une composition tensioactive suivant les revendications 1 et 2, dans la cosmétique des cheveux.

6. Utilisation d'une composition tensioactive suivant les revendications 1 et 2, dans des fixateurs, rinçages, pulvérisations, produits pour ondulation permanente ou produits de teinture destinés aux cheveux.
